(19) Europäisches Patentamt
European Patent Office
Office européen des brevets

(11) Publication number : 0 451 022 A1

(12) EUROPEAN PATENT APPLICATION

(21) Application number : 91400825.5

(22) Date of filing : 27.03.91

(51) Int. Cl.$^5$ : C07C 217/74, A61K 31/135

(30) Priority : 30.03.90 EP 90400887

(43) Date of publication of application :
09.10.91 Bulletin 91/41

(84) Designated Contracting States :
AT BE CH DE DK ES FR GB GR IT LI LU NL SE

(71) Applicant : MERRELL DOW PHARMACEUTICALS INC.
2110 East Galbraith Road
Cincinnati Ohio 45215-6300 (US)

(72) Inventor : Hibert, Marcel
Domaine des Grands-Prés, 21, rue Alfred Kastler
F-67114 - Eschau (FR)
Inventor : Zimmermann, André
3-rue des Cerisiers
F-67200 - Achenheim (FR)

(74) Representative : Gillard, Marie-Louise et al
Cabinet Beau de Loménie 55, Rue d'Amsterdam
F-75008 Paris (FR)

(54) **Novel compounds for the treatment of migraine.**

(57) This invention relates to the use of 8-methoxy-2-amino-1,2,3,4-tetrahydronaphthalenes in the treatment of migraine and related disorders.

EP 0 451 022 A1

This invention relates to the use of 8-methoxy-2-amino-1,2,3,4-tetrahydronaphthalenes in the treatment of migraine and related disorders.

A wide variety of 1,2,3,4-tetrahydro-2-naphthalamines have been described in PCT/SE81/00155 having the International Publication number WO 81/03491 of 10 December 1981, it being stated therein that the compounds have therapeutic activity in the central nervous system having an effect on the 5-hydroxy-tryptamine neurones in man and are useful in treating disorders in the CNS such as depression and sexual disturbances, and are also potentially useful in the control of pain and senile disturbances of movement and mental function wherein 5-HT has been shown to be involved.

This invention relates to compounds which, although they are generically embraced within the scope of the above-mentioned PCT application (as well as in Ames et al, J. Chem. Soc. 1965, 20, 36-41), there is no teaching for their use as therapeutic agents for the treatment of migraine. More specifically, this invention relates to the specific compounds 2-amino-8-methoxy-1,2,3,4-tetrahydronaphthalene and 2-dimethyl-amino-8-methoxy-1,2,3,4-tetrahydronaphthalene, the individual and mixtures of their enantiomeric forms, and to the pharmaceutically acceptable salts thereof for the use of these compounds in the treatment of migraine. Additionally, this invention includes the use of these compounds for the preparation of medicaments useful in treating migraine.

Expressed otherwise, the compounds of this invention are compounds of the formula

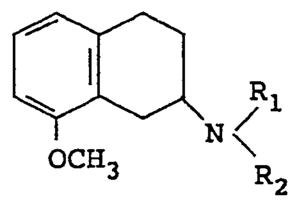

I

wherein both of $R_1$ and $R_2$ are H or both are methyl, including the isolated or mixtures of their enantiomers and the pharmaceutically acceptable salts thereof.

Both organic and inorganic acids can be employed to form non-toxic pharmaceutically acceptable acid addition salts of the compounds of this invention. Illustrative acids are sulfuric, nitric, phosphoric, hydrochloric, citric, acetic, lactic, tartaric, palmoic, ethanedisulfonic, sulfamic, succinic, cyclohexylsulfamic, fumaric, maleic and benzoic acid. These salts are readily prepared by methods known in the art.

The compounds of this invention contain an asymmetric carbon atom in the aliphatic ring moiety, i.e., the ring carbon atom adjacent to the nitrogen atom. The therapeutic properties of the compounds may to a greater or lesser degree be ascribed to either or both of the two enantiomers occurring. Thus the pure enantiomers as well as mixtures thereof are within the scope of the invention. The resolution of the enantiomers may be effected by such techniques as fractional distillation, chromatographic methods and preferably by enantioselective crystallization using optically pure acids such as (+) or (-) binaphthyl phosphonic acid. Typically, the amine and optically pure binaphthyl phosphonic acid dissolved in acetone or warm isopropanol are mixed and maintained between 4 and 25°C until crystallization of one of the two diastereoisomeric salts. The solid is separated and recrytallized and the optically active free amine is recovered by extraction with ether from an aqueous solution of potassium carbonate. The other enantiomer is recovered from the mother liquors, basified and recrystallized with the other enantiomer of the binaphthyl phosphonic acid.

The compounds of this invention may be prepared by the methods of the following examples.

EXAMPLE 1

**2-Amino-8-methoxy-1,2,3,4-tetrahydronaphthalene**

STEP A:

8-Methoxy-2-benzylamino-1,2,3,4-tetrahydronaphthalene

Under nitrogen, a solution of 8-methoxy-2-tetralone (1.76 g, 10 mmol), benzylamine (30 mmol), acetic acid (1.8 g, 1.7 ml, 30 mmol) and ethanol (25 ml) is allowed to stand for 2 hours over 3A molecular sieves (2.5 g). The solution is then decanted into a hydrogenation flask, and the sieves rinsed with ethanol. To the combined solution is added $PtO_2$ (150 mg) and subsequent hydrogenation is performed at atmospheric pressure. After filtration (to remove the catalyst) and evaporation of solvents *in vacuo*, the crude amine product is purified by filtration through a short alumina column (Woelm, Akt 1) using 3:1 n-hexane:ethyl acetate as eluant. The hydrochloride salt is prepared and recrystallized (methanol/ethyl acetate) to give needles, m.p. 218-219°C (62%).

STEP B:

A reaction mixture is prepared containing 8-methoxy-2-benzyl-amino-1,2,3,4-tetrahydronaphthalene (2.0 g, 6.6 mmol), Pearlman's catalyst ($Pd(OH)_2/C$, 1.0 g) and ethanol, and hydrogenated under pressure (Parr, 40 psi) for 3 hours. The resulting reaction mixture is filtered through celite and the filtrate evaporated *in vacuo* to give a dark-brown solid. The hydrochloride salt, obtained by treatment with ethanolic hydrochloride, is decolorized with charcoal and recrystallized from water to give the desired product as fine needles, m.p. 273-274°C.

Although the foregoing example illustrates the removal of a benzyl protecting group for the 2-position amine moiety, any equivalently functioning amino protecting group may similarly be utilized, as well as other standard methods for the removal of any such amino protecting groups.

EXAMPLE 2

**2-Dimethylamino-8-methoxytetralin, hydrochloride**

8-Methoxy-2-aminotetralin (0.005 mol) is added to 10 ml of methanol which contained 0.025 mol of 37% aqueous formaldehyde and 0.015 mol of sodium cyanoborohydride The mixture is stirred at room temperature under an argon atmosphere and brought to pH 6 by addition of glacial acetic acid. The mixture is stirred overnight, the volatiles are removed under vacuum and the residue is basified with 2N sodium hydroxyde. The aqueous solution is extracted with dichloromethane. The organic phase is dried over sodium sulfate and evaporated to dryness to yield the crude amine as an oil. This is converted to its hydrochloride salt, which recrystallizes from 2-isopropanol-ether to give the title compound, m.p.: 240°C.

In addition to the use of a formaldehyde-$Na(CN)BH_3$ mixture, the alkylation to form a dimethyl amino moiety may be effected using a formaldehyde-formic acid mixture. Also the alkylation may be effected by reaction with a methyl halide or tosylate as well as other standard procedures also well known in the art.

The compounds of this invention are potent and selective $5\text{-}HT_1$-like agonists useful in treating and preventing reoccurrence of migraine. Using standard *in vitro* and *in vivo* laboratory assay methodology for testing compounds for their potential use in the treatment of migraine, as well as by comparison with compounds known or reportedly found to have beneficial therapeutic effects in patients suffering from migraine (and related disorders such as cluster headache) suitable daily doses of the compounds of this invention for the treatment of migraine will be about 50 to 500 mg for enteral administration and about 0.1 to 100 mg for parenteral administration, said dosage based upon administration to a 70 kilogram bodyweight man. Of course it is to be appreciated that it may be necessary to make routine variations in the daily dosages depending upon such factors as the age and weight of the patient, the patient's general health condition, as well as the severity of the condition to be treated.

In practice the compounds of the present invention will normally be administerred orally, rectally, or by injection, in the form of pharmaceutical preparations comprising the active ingredient either as a free base or as a pharmaceutically acceptable non-toxic, acid addition salt, e.g., the hydrochloride, lactate, acetate, sulfamate, and the like, in association with a pharmaceutically acceptable carrier.

The carrier may be a solid, semisolid or liquid diluent or capsule. These pharmaceutical preparations constitute a further aspect of this invention. Usually the active substance will constitute between 0.1 and 99% by weight of the preparation, more specifically between 0.5 and 20% by weight for preparations intended for injection and between 0.2 and 50% by weight for preparations suitable for oral administration.

Pharmaceutical preparations containing a compound of the invention may preferably contain between 2 and 50% by weight of the active substance, in such preparations the selected compound may be mixed with a solid fine grain carrier, e.g., lactose, saccharose, sorbitol, mannitol, starches such as potato starch, corn starch or amylopectin, cellulose derivatives, or gelatin and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g., gum arabic, gelatin, talcum, titanium dioxide, and the like. Alternatively the tablet can be coated with a lacquer dissolved in a readily volatile organic solvent or mixture of organic solvents. Dyestuffs may be added to these coatings in order to readily distinguish between tablets containing different active substances or different amounts of the active compound.

For the preparation of soft gelatin capsules (pearl-shaped closed capsules) consisting of gelatin and, for example, glycerol, or similar closed capsules, the active substance may be admixed with a vegetable oil. Hard gelatin capsules may contain granulates of the active substance in combination with solid, fine grain carriers such as lactose, saccharose, sorbitol, mannitol, starches (e.g., potato starch, corn starch or amylopectin) cellulose derivatives or gelatin.

Liquid preparations for oral application may be in the form of syrups or suspensions, for example, solutions containing from about 0.2% to about 20% by weight of the active substance herein described, the balance being sugar and a mixture of ethanol, water, glycerol and propyleneglycol. Optionally such liquid preparations may contain coloring agents, flavoring agents, saccharine and carboxymethylcellulose as a thickening agent.

Solutions for parenteral applications by injection can be prepared in an aqueous solution of a water-soluble pharmaceutically acceptable salt of the active substance, preferably in a concentration of from about 0.5% to about 10% by weight. These solutions may also contain stabilizing agents and/or buffering agents and may conveniently be provided in various dosage unit ampoules.

The following examples illustrate how the compounds of this invention may be formulated into pharmaceutical preparations.

Preparation of tablets

The active substance (0.5 kg) is mixed with 0.2 kg of silicic acid of Aerosil®. Potato starch (0.45 kg) and lactose (0.5 kg) are mixed therewith and the mixture is moistened with a starch paste prepared from 50 g of potato starch and distilled water, whereupon the mixture is granulated through a sieve. The granulate is dried and sieved, whereupon 20 g of magnesium stearate are mixed into it. Finally the mixture is pressed into tablets each weighing 172 mg.

Capsules

| | mg/capsules |
|---|---|
| Active ingredient | 10.0 |
| *Starch 1500 | 89.0 |
| Magnesium Stearate BP | 1.0 |
| Fill Weight | 100.0 |

**A form of directly compressible starch*

The active ingredient is sieved and blended with the excipients. The mix is filled into size No.2 hard gelatin capsules using suitable machinery. Other doses may be prepared by altering the fill weight and if necessary changing the capsule size to accomodate the alteration.

Syrup

| | mg/5ml dose |
|---|---|
| Active ingredient | 10.0 |
| Sucrose BP | 2,750.0 |
| Glycerine BP | 500.0 |
| Buffer | as required |
| Flavor | as required |
| Color | as required |
| Preservative | as required |
| Distilled water to | 5.0 ml |

The active ingredient buffer, flavor, color and preservative are dissolved in some of the water and the glycerine is added. The remainder of the water is heated to dissolve the sucrose and is then cooled. The two solutions are combined, adjusted to volume and mixed. The syrup produced is clarified by filtration.

Suppositories

| | | |
|---|---|---|
| Active ingredient | 10.0 | mg |
| *Witepsol H15 to | 1.0 | g |

**A proprietary grade of Adeps Solidus Ph Eur*

A suspension of the active ingredient in molten Witepsol is prepared and filled, using suitable machinery, into 1 g size suppository moulds.

Injection for intravenous administration

| | % w/v |
|---|---|
| Active ingredient | 0.2 |
| Sodium chloride BP | as required |
| Water for injection BP to | 100.0 |

Sodium chloride may be added to adjust the tonicity of the solution and the Ph may be adjusted using acid or alkali, to that of optimum stability and/or to facilitate solution of the active ingredient. Alternatively suitable buffer salts may be used.

The solution is prepared, clarified and filled into appropriate size ampoules sealed by fusion of the glass. The injection is sterilized by heating in an autoclave using one of the acceptable cycles. Alternatively the solution may be sterilized by filtration and filled into sterile ampoules under aseptic conditions. The solution may be packed under an inert atmo sphere of nitrogen or other suitable gas.

Inhalation cartridges

| | mg/cartridge |
|---|---|
| Active ingredient micronized | 1.0 |
| Lactose BP | 39.0 |

The active ingredient is micronized (Micronizer is a Registered Trademark) in a fluid energy mill to a fine particle size range prior to blending with normal tabletting grade lactose in a high energy mixer. The powder blend is filled into No.3 hard gelatin capsules on a suitable encapsulating machine. The contents of the cartridges are administered using a powder inhaler such as the Glaxo Rotahaler®.

Metered dose pressurised aerosol

| | mg/metered dose | per can |
|---|---|---|
| Active ingredient micronized | 0.50 | 120.00 mg |
| Oleic Acid BP | 0.05 | 12.00 mg |
| Trichlorofluoromethane BP | 22.25 | 5.34 mg |
| Dichlorofluoromethane BP | 62.20 | 14.92 g |

The active ingredient is micronized in a fluid energy mill to a fine particle size range. The oleic acid is mixed with the trichlorofluoromethane at a temperature of 10-15°C and the pulverized drug is mixed into the solution with a high shear mixer. The suspension is metered into aluminium aerosol cans and suitable metering valves delivering a metered amount of 85 mg of suspension are crimped onto the cans and the dichlorofluoromethane is pressure filled into the cans through the valves.

## Claims

1. The use of a compound of the formula

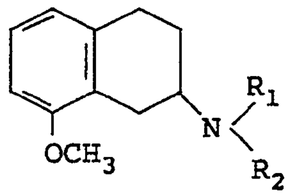

I

wherein both of $R_1$ and $R_2$ are H or both are methyl, including the isolated or mixtures of their enantiomers and the pharmaceutically acceptable salts thereof, for the preparation of medicaments useful in treating migraine.

European Patent Office

# EUROPEAN SEARCH REPORT

Application Number

EP 91 40 0825

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 253 257 (BAYER AG) * Example 1 * --- | 1 | C 07 C 217/74<br>A 61 K 31/135 |
| A | EP-A-0 334 538 (THE UPJOHN CO.) * Page 7, line 43; chart 1 * --- | 1 | |
| Y | JOURNAL OF MEDICINAL CHEMISTRY, vol. 32, no. 4, April 1989, pages 779-783, American Chemical Society, Washington, DC, US; L. BJÖRK et al.: "Resolved N,N-dialkylated 2-amino-hydroxytetralins: Stereoselective interactions with 5-HT1A receptors in the brain" * Scheme I; pages 779-780 * --- | 1 | |
| A | JOURNAL OF MEDICINAL CHEMISTRY, vol. 32, no. 2, February 1989, pages 478-486, American Chemical Society, Washington, DC, US; Q. JE et al.: "Conformationally restricted and conformationally defined tyramine analogues as inhibitors of phenylethanolamine N-methyltransferase" * Compound 26 * --- | 1 | **TECHNICAL FIELDS SEARCHED (Int. Cl.5)**<br>C 07 C 217/00<br>A 61 K 31/00 |
| Y | JOURNAL OF MEDICINAL CHEMISTRY, vol. 27, no. 1, January 1984, pages 45-51, American Chemical Society, Washington, DC, US; L.-E. ARVIDSSON et al.: "8-Hydroxy-2-(alkylamino)tetralins and related compounds as central 5-hydroxy-tryptamine receptor agonists" * Compound 14 * --- -/- | 1 | |

The present search report has been drawn up for all claims

| Place of search | Date of completion of the search | Examiner |
|---|---|---|
| THE HAGUE | 20-06-1991 | SANCHEZ Y GARCIA J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 91 40 0825

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JORNAL OF NEUROCHEMISTRY, vol. 49, no. 2, 1987, pages 373-380, International Society for Neurochemistry, New York, US; M.B. EMERIT et al.: "Identification of the 5-HT1A receptor binding subunit in rat brain membranes using the photoaffinity probe [3H]8-methoxy-2-[N-n-propyl, N-3-(2-nitro-4-azidophenyl)aminopropyl] Aminotetralin" * Page 374: "Materials and methods" * --- | 1 | |
| Y | TRENDS IN PHARMACOLOGICAL SCIENCES, vol. 10, 1989, pages 200-204, Elsevier Science Publishers Ltd, London, GB; P.R. SAXENA et al.: "5-HT1-like receptor agonists and the pathophysiology of migraine" * Whole article * --- | | |
| Y | CEPHALAGIA, suppl. 9, 1989, pages 15-22, Oslo, NO; P.R. SAXENA et al.: "Characterization of 5-hydroxytruptamine receptors in the cranial vasculture" * Whole article * --- | 1 | TECHNICAL FIELDS SEARCHED (Int. Cl.5) |
| Y | ANNALS OF NEUROLOGY, vol. 23, no. 5, May 1988, pages 500-504, American Neurological Association, Tokyo, JP; S.J. PEROUTKA: "Antimigraine drug interactions with serotonin receptor subtypes in human brain" * Whole article * --- | 1 | |
| A,D | WO-A-8 103 491 (ASTRA LÄKEMEDEL AB) * Claims 1,2 * ----- | 1 | |

The present search report has been drawn up for all claims

| Place of search | Date of completion of the search | Examiner |
|---|---|---|
| THE HAGUE | 20-06-1991 | SANCHEZ Y GARCIA J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)